Europäisches Patentamt

European Patent Office

Office européen des brevets

(11) Publication number: **0 267 521**
**A2**

## (12) EUROPEAN PATENT APPLICATION

(21) Application number: **87116142.8**

(22) Date of filing: **03.11.87**

(51) Int. Cl.⁴: **C12Q 1/68** , G01N 33/543 , G01N 33/545

(30) Priority: **12.11.86 US 929958**

(43) Date of publication of application:
**18.05.88 Bulletin 88/20**

(84) Designated Contracting States:
**AT BE CH DE ES FR GB IT LI LU NL SE**

(71) Applicant: **Molecular Diagnostics, Inc.**
**400 Morgan Lane**
**West Haven, CT.06516(US)**

(72) Inventor: **Dattagupta, Nanibhushan, Dr.**
**470 Prospect Street**
**New Haven, CT 06511(US)**
Inventor: **Busse, Wolf Dieter, Dr.**
**Pahlkestrasse 65**
**D-5600 Wuppertal 1(DE)**

(74) Representative: **Dänner, Klaus, Dr. et al**
**c/o Bayer AG Konzernverwaltung RP**
**Patentabteilung**
**D-5090 Leverkusen 1 Bayerwerk(DE)**

(54) **Method for the detection of nucleic acid hybrids.**

(57) A method for detecting the presence of nucleic acid hybridization comprising

(a) immobilizing onto a solid support a first nucleic acid,

(b) contacting under hybridizing conditions the immobilized first nucleic acid of step (a) with a labeled second nucleic acid which is suspected of being complementary to said first nucleic acid under hybridizing conditions, and

(c) contacting the resultant material from step (b) with a metal coated with a protein specifically bindable with the label, the metal being capable of absorbing proteins.

EP 0 267 521 A2

## METHOD FOR THE DETECTION OF NUCLEIC ACID HYBRIDS

### BACKGROUND OF THE INVENTION

#### Field of the Invention

The present invention concerns a non-radioactive method for detection of immobilized nucleic acid hybrids. More particularly, the present invention relates to a method for detecting nucleic acid hybrids on nitrocellulose paper using a metal coated with a protein, e.g., anti-biotin antibody coated gold particles.

#### Background Information

Nucleic acid hybridization is presently the most powerful technique for the determination of genetic structure. It is used extensively for research, and DNA probe technology is playing an ever increasing role in prenatal diagnosis of hereditary dissorders (E. M. Rubin and Y. W. Kan, The Lancet, 75-77 (1985); M. E. Pembrey, J. Appl. Med., 10, 213-221, (1984)) and in other clinical assays (K. -O. Habermehl, ed., Rapid Methods and Automation in Microbiology and Immunology, Springer-Verlag, Berlin, pp. 30-82, (1985); R. K. Saiki, S. Scharf, F. Faloona, K. B. Mullis, G. T. Horn and N. A. Erlich, Science, 230 , 1350-1354, (1985) and N. Kalsheker, D. Chiswell, A. Markham, A. Imam, S. Wallis, R. Williamson and S. E. Humphries, Ann. Clin. Biochem., 22, 25-32, (1985)).

The basic method requires that the DNA probe be labeled with a detectable marker for determination of the hybrid. The most sensitive labeling method uses radioisotope phosphorous-32 as the detectable label. Because of the hazard associated with radioisotopes and the relative instability of phosphorous-32, several other labeling methods have been developed (A. C. Forster, J. L. McInnes, D. C. Skingle and H. R. Symons, Nucleic Acids Res., 13, 745-761, (1985); P. R. Langer, A. A. Waldrop and D. C. Ward, Proc. Natl. Acad. Sci. USA, 78, 6633-6637, (1981); J. J. Leary, D. J. Brigati and D. C. Ward, Proc. Natl. Acad. Sci. USA, 80, 4045-4049, (1983); P. Tchen, R. P. P. Fuchs, E. Sage and M. Leng, Proc. Natl. Acad. Sci. USA, 81, 3466-3470 (1984) and M. Renz and C. Kurz, Nucleis Acids Res., 12, 3435-3444, (1984)).

The most successful alternative uses biotin as the label. The label is most often introduced via a biotinylated nucleoside triphosphate and nick translation (Langer et al, supra; Leary et al, supra). Following hybridization, the hapten (biotin) is detected colorimetrically using a biotin binding protein (avidin or streptavidin) to which an enzyme is coupled. Incubation with a substrate results in visualization of the biotinylated hybrids. The most sensitive of such systems are commercially available (Enzo, BRL, Amersham).

Recently a similar enzyme coupled system using chemiluminescence detection has been described (J. A. Matthews, B. Armaiti, C. Hynds and L. J. Kricka, Anal. Biochem., 151, 205-209, (1985)).

All of the above described systems require enzymes for their sensitivity and as would be expected background problems, reagent stability and sensitivity make these systems non-ideal in clinical laboratories.

Heretofore, gold particles have been used in the staining of and the immunodetection of proteins transferred to or immobilized onto nitrocellulose paper (M. Moeremans, G. Daneels, A. Van Dijck, G. Langanger and J. De Mey, J. Immunol. Methods, 74, 353-360, (1984); B. Surek and E. Latzko, Biochem. Biophys. Res. Comm., 121, 284-289, (1984) and Y. Hsu, Anal. Biochem., 142, 221-225, (1984)). However, gold particles have not heretofore been utilized in nucleic acid hybridizations carried out on nitrocellulose papers.

It would be advantageous to have an efficient, sensitive, non-radioactive and non-enzymatic detection system for nucleic acid hybrids.

### SUMMARY OF THE INVENTION

It is an object of the present invention to provide a non-radioactive, non-enzymatic, highly efficient and sensitive system for detection of nucleic acid hybrids. This object and other objects and advantages are afforded by the present invention.

The present invention concerns a method for detecting the presence of nucleic acid hybridization comprising

(a) immobilizing onto a solid support a first nucleic acid,

(b) contacting the immobilized first nucleic acid of step (a) under hybridizing conditions with a labeled second nucleic acid which is suspected of being complementary to said first nucleic acid, and

(c) contacting the resultant material from step (b) with a metal coated with a protein specifically bindable with the label, the metal being capable of absorbing proteins.


## BRIEF DESCRIPTION OF THE DRAWINGS

Fig. 1 depicts detection of hybridized DNA on nitrocellulose filters. Panel (a) is for a goat anti-biotin antibody labeled gold probe with silver enhancement according to the present invention and panel (b) is for a BRL colorimetric DNA detection system (prior art).

Fig. 2 is a densitometric scan of hybridized filters shown in Fig. 1 for (a) a gold probe detection according to the invention and (b) a BRL detection (prior art).

Fig 3 is a graph of relative signal intensity vs. immobilized DNA concentration for gold probe detection (x) according to the present invention and BRL detection (o) (prior art).


## DETAILED DESCRIPTION OF THE INVENTION

The nucleic acid component can be single or double stranded DNA or RNA or fragments thereof such as are produced by restriction enzymes or even relatively short oligomers.

If the nucleic acids are in double stranded form they have to be denatured before hybridization. Denaturation can be carried out by heating, e.g., in a boiling water bath or by alkali and acid or by an organic solvent, e.g., dimethylsufoxide. If acid or alkali denaturation is carried out, neutralization prior to hybridization is conducted by adding acid (for alkali) or alkali (for acid). Heat denatured samples are usually quenched in ice.

Useful solid supports are well known in the art and include those which bind nucleic acids either covalently or noncovalently. Noncovalent supports which are generally understood to involve hydrophobic bonding include naturally occurring and synthetic polymeric materials, such as nitrocellulose, derivatized nylon and fluorinated polyhydrocarbons, in a variety of forms such as filters, beads or solid sheets. Covalent binding supports (in the form of filters, beads or solid sheets, just to mention a few) are also useful and comprise materials having chemically reactive groups or groups such as dichlorotriazine, diazobenzyloxymethyl, and the like, which can be activated for binding to polynucleotides.

Typical hybridization conditions include the use of the following:

(1) a buffer, e.g., 5$^\times$ SSC (44 grams NaCl, 22 grams Na citrate, approximately 0.25 ml HCl in a liter of water),

(2) an organic solvent, e.g., 0-50% formamide,

(3) Denhardt's solution (0.02% w/v of each of "FICOLL 400", polyvinylpyrrolidine and BSA),

(4) Accelerators, e.g., 10% dextran sulfate or polyethylene glycol.

A specific composition is described in the examples hereinbelow.

The label is to be compatible with the protein. Non-limiting examples of label/protein combinations that can be employed in the present invention include the following:

| label | protein |
|---|---|
| hapten | antibody |
| antigen | antibody |
| biotin | avidin |
| FAD | glucose oxidase |
| biotin | anti-biotin antibodies |

Non-limiting examples of metals for use in the present invention include gold, silver, iron, nickel, cobalt and mercury. Gold is particularly preferred.

The present invention has the following advantages:

(1) high efficient (one hour as opposed to up to four hours),

3

(2) very sensitive,

(3) does not require the use of radioactive moieties or enzymes,

(4) relatively simple to carry out.

The invention will now be described with reference to the following non-limiting examples.

## EXAMPLES

### Materials

The materials utilized in the examples described hereinbelow were obtained by the following sources: Goat anti-biotin antibody was purchased from Sigma Chemical Company (St. Louis, Missouri, U.S.A.). G-20 Colloidal gold sol and silver enhancement kits were purchased from Janssen Life Sciences Products (Piscataway, New Jersey, U.S.A.). Phage lambda DNA, a colorimetric DNA detection system and a Hybri-Slot Manifold were purchased from Bethesda Research Laboratories ("BRL") (Gaithersburg, Maryland, U.S.A.). The nitrocellulose filters used were of 0.2 μm pore size and were obtained from Schleicher and Schuell Inc. (Keene, New Hampshire, U.S.A.). The photochemical labeling reagent, N-(4-azido-2-nitrophenyl)-N'-(N-d-biotinyl-3-aminopropyl)-N'-methyl-1-1,3-propanediamine (Photobiotin), was prepared by a modification of the method of Forster et al, supra.

### Example 1: Preparation of Immunogold Probe

The probe was prepared essentially as described in the Janssen instruction booklet supplied with the colloidal gold sols (W. D. Geoghegan and G. A. Ackerman, J. Histochem. Cytochem., 25, 1187-1200, (1977) and W. P. Faulk and G. M. Taylor, Immunocytochemistry, 8, 1080-1083, (1971)).

Affinity isolated goat anti-biotin antibody was dialyzed overnight against 2 mM sodium tetraborate, pH 9. The antibody solution was then centrifuged at 100,000 $^{\times}$g for one hour and the upper three-quarters of the supernatant was retained. The antibody concentration was determined using an extinction coefficient of 14, and diluted in 2 mM borate to give a concentration of 0.2 mg/ml.

To determine the minimal protecting amount of protein (the optimum concentration of antibody for adsorption to the colloidal gold) (Geoghegan et al supra; Faulk et al supra and J. Roth, Techniques in Immunocytochemistry, (G. R. Bullock and P. Petrusz, eds.), Vol. 2, pp. 217-284, Academic Press, London, (1983)) ten linear dilutions of the antibody were prepared from 20 μg to 2 μg in 100 μl of 2 mM borate pH 9. To each antibody dilution was added 1 ml of G-20 colloidal gold sol pre-adjusted to pH 9 with 0.1 M K₂CO₃ and the resultant mixture was vortexed. Following a two minute adsorption period, 100 μl of 10% NaCl was added to each tube and the O.D. at 580 mn was determined. Unstabilized (unprotected) colloidal gold turned from red to blue upon addition of salt and the minimal protecting amount of antibody was the lowest concentration that prevents this color change after addition of the salt. The minimal protecting amount of goat anti-biotin antibody was found to be 8 μg/ml G-20 colloidal gold sol.

The probe was thus prepared by mixing the goat anti-biotin antibody with the pH adjusted gold sol at a concentration of 8 μg/ml. After a two minute adsorption period 10% BSA in PBS adjusted to pH 8.5 with 0.1 M K₂CO₃ was added to give a final concentration of 1% BSA (w/v). the probe was washed three times by centrifuging at 12,000 $^{\times}$ g for 30 minutes and resuspending the loose pellet to the original volume with 1% BSA in PBS (pH 8.5). Following the final wash, the loose pellet was resuspended and brought to an O.D. at 520 mn of 5.0. The probe was stored at 4°C and diluted with 0.05% BSA in PBS to an O.D. at 520 nm of 0.5 prior to use.

### Example 2: Immobilization of DNA to Nitrocellulose Filter

A nitrocellulose filter was hydrated in distilled water and then soaked in 5 $^{\times}$ SSC for 15 minutes. The filter was then air dried and clamped into a Hybi-Slot Manifold. Dilutions of phage lambda and phage M13mp7 DNA were made in 1 M NaCl, 0.1 m NaOH and 5 mM EDTA. The diluted samples were heated at 100°C for 5 minutes and then neutralized with an equal volume of 2 M ammonium acetate. Two μl of the appropriate DNA dilutions were pipetted into each sample position and following a 15 minute period, the filter was removed and baked at 80°C for 45 minutes. Filters thus prepared were stored in sealed plastic bags at 4°C.

## Example 3: DNA Labeling

Phage lambda DNA was labeled with biotin using photobiotin, a photchemical coupling reagent. Lambda DNA (250 μg/ml in a TE buffer was mixed with an equal amount (w/w) of photobiotin in distilled water and illuminated for 1 hour in a beam of a "KODAK" slide projector (3 cm from lens), fitted with a 300W tungsten-halogen lamp. The labeled DNA was then recovered by ethanol precipitation.

## Example 4: Hybridization

Nitrocellulose filters containing the immobilized samples of lambda and M13mp7 DNA were prehybridized in 10 ml of 45% formamide, 50 mM sodium phosphate pH 6.5, 5 $\times$ SSC, 10 $\times$ Denhardts solution and 250 μg/ml denatured salmon sperm DNA for 4 hours at 42°C. Hybridization was carried out overnight at 42°C in sealed plastic bags containing 5 ml of 45% formamide, 20 mM sodium phosphate pH 6.5, 5 $\times$ SSC, 5 $\times$ Denhardts solution, 100 μg/ml denatured salmon sperm DNA and 250 ng/ml of boiled (10 minutes) biotinylated lambda DNA.

Following hybridization the filters were washed 2 $\times$ 5 minutes in 2 $\times$ SSC, 0.1% SDS, 2 $\times$ 5 minutes in diluted SSC (1 part SSC and 4 parts H$_2$O), 0.1% SDS and 2 $\times$ 15 minutes in diluted SSC (1 part SSC and 6 parts H$_2$O), 0.1% SDS. All wash steps were performed at 50°C. The filters were then blocked in 0.1 M Tris/HCl pH 7.5, 0.1 M NaCl, 2 mM MgCl$_2$ containing 3% BSA for one hour at room temperature.

## Example 5: Detection of Hybridized DNA

Two methods of detecting biotinylated hybridized DNA were employed. the colorimetric DNA detection system of BRL was used according to the instructions supplied with the BRL kit. Briefly, the blocked filters were incubated with streptavidin, washed, incubated with biotinylated poly-alkaline phosphatase, washed and visualized with a dye solution containing nitro-blue tetrazolium and 5-bromo-4-chloro-3-indolyl phosphate. Color development was allowed to proceed for three hours.

Detection with the anti-biotin gold probe according to the present invention was achieved by incubating the blocked filters with a 1/10 dilution of the prepared probe for one hour at room temperature. The filters were then washed for 2 $\times$ 3 minutes in distilled water and silver enhanced using a Janssen silver enhancement kit, according to the supplied instructions (Moeremans et al, supra).

The filters were scanned using a Joyce Loebl Chromoscan 3 scanning densitometer in the reflection mode. The unit was equipped with a high power arc lamp and no optical filter was used during the scan.

DNA hybridization assays on nitrocellulose filters were carried out in order to evaluate a direct method of detection of hybridized biotinylated DNA using an immuno-gold probe. A quantitative comparison was made with the BRL colorimetric DNA detection system, a widely used alternative to detection by isotopic labeling.

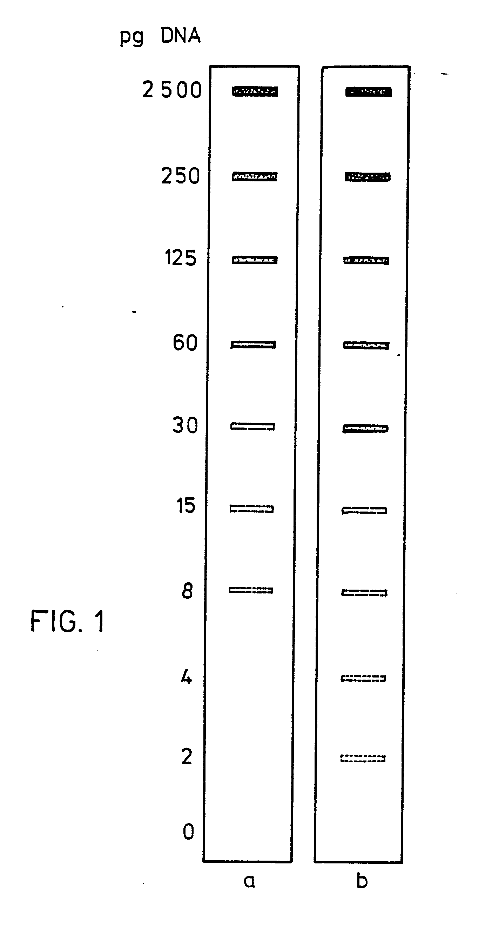

Onto duplicate sets of two nitrocellulose filters (2 μl) were immobilized phage lambda and phage M13mp7 DNA in the amounts indicated in Fig. 1. Following overnight hybridization with biotinylated lambda DNA, one set of filters was detected using the colorimetric BRL system and the other using the immuno-gold probe (goat anti-biotin antibody adsorbed to 20 nm diameter gold particles). Filters containing immobilized M13mp7 DNA were included as a control for non-specific hybridization. Neither method of detection revealed any signal when these filters were hybridized with biotinylated lambda DNA.

The conditions used for immobilization of DNA to nitrocellulose, prehybridization and hybridization were determined to be optimal for maximum sensitivity by the BRL detection system.

Fig. 1 shows the assay results obtained using both methods of detection. The conditions used for detection by gold probe are described hereinabove. Concentrations higher than 0.05% BSA in the gold probe incubation buffer resulted in decreased sensitivity and lower concentrations of BSA gave a high background resulting from non-specific adsorption of th gold probe to the nitrocellulose. The presence of "TWEEN", a detergent commonly used in immunoassays, during incubation of the filter with the gold probe was found to seriously reduce the signal obtained.

The nitrocellulose filters were incubated for One hour with the gold probe and periods of up to 3 hours gave no increase in sensitivity. However, longer periods using the gold probe at the concentration described, resulted in increased background.

Following incubation of the nitrocellulose filters with the gold probe, biotinylated hybridized to 30 pg of immobilized DNA could be detected. Detection down to 8 pg was achieved following silver enhancement, after which the hybridized DNA was visualized as black spots, contrasting well against the white background. Prior to silver enhancement, the filters were washed for 2 × 3 minutes in distilled water. Shorter wash periods may result in a higher background following enhancement and longer wash periods may result in decreased signal intensity. The gold probe, if stored at 4°C with 0.01% sodium azide, can be re-used several times before any deleterious effects on sensitivity can be seen.

Filters detected using the BRL system were allowed to develop in the dye solution for three hours. It was found that this length of time was optimal for maximum contrast between signal and background.

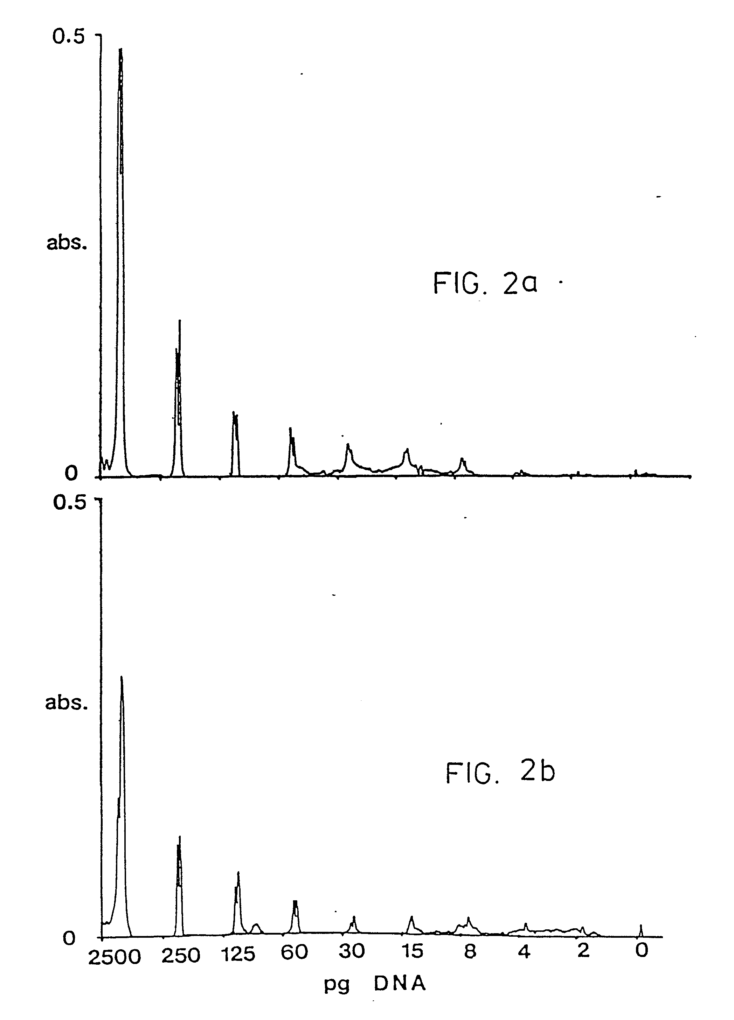

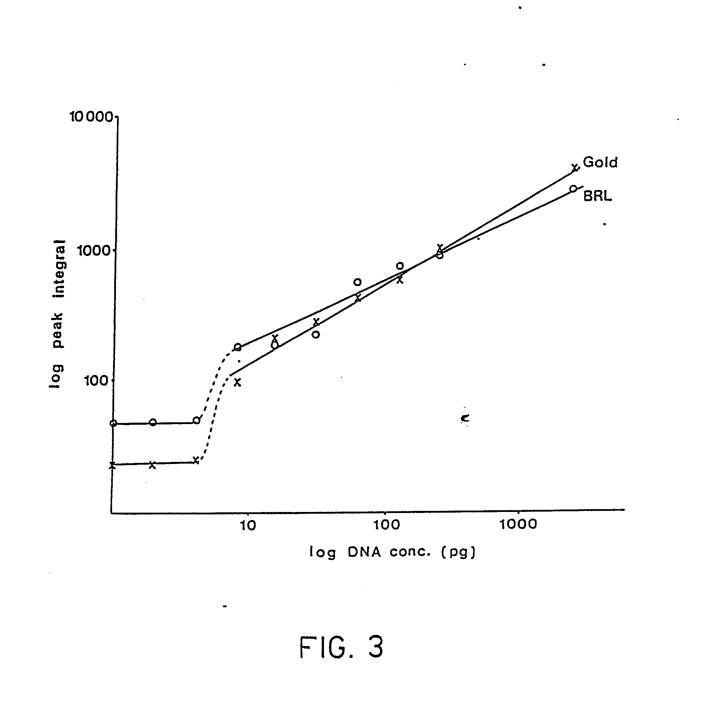

The results shown in Fig. 1 are quantitatively depicted in Fig. 2 wherein the filters were scanned by measuring reflected light without an optical filter at an aperture width of 0.1 mm. Fig. 3 shows that the relationship between signal intensity and applied DNA concentration appears similar for both detection methods and that both are comparable with regard to sensitivity, detecting down to 8 pg of applied DNA. In Fig. 3 integrated areas of peaks from the densitometric scan of filters are depicted as a function of applied DNA.

Filters detected using the BRL method, however, consistently gave a higher background than those detected using the gold probe, and without any means of quantifying the results, it was often difficult to determing a positive signal over background at the lower applied DNA concentrations.

It is evident from the above results that a non-enzymatic biotin detection system with gold particles is as sensitive as an enzyme coupled system. The gold probe detection method, as well as being considerably faster than enzymatic detection methods, is much less labor intensive. It consists simply of one incubation step followed by a 10 minute silver enhancement step. During the gold probe incubation, biotinylated DNA hybrids with the higher concentrations of immobilized DNA can be seen after only 10 minutes.

It will be appreciated that the instant specification and claims are set forth by way of illustration and not limitation and that various modifications and changes may be made without departing from the spirit and scope of the present invention.


## Claims

1. A method for detecting the presence of nucleic acid hybridization comprising
    (a) immobilizing onto a solid support a first nucleic acid,
    (b) contacting under hybridizing conditions the immobilized first nucleic acid of step (a) with a labeled second nucleic acid which is suspected of being complementary to said first nucleic acid under hybridizing conditions, and
    (c) contacting the resultant material from step (b) with a metal coated with a protein specifically bindable with the label, said metal being capable of absorbing proteins.

2. A method according to claim 1, wherein said metal is selected from the group consisting of gold, silver, cobalt, iron, nickel and mercury.

3. A method according to claim 2, wherein the metal is gold.

4. A method according to any of claims 1 to 3, wherein the label for the second nucleic acid is selected from the group hapten, antigen, biotin and FAD.

5. A method according to any of claims 1 to 4, wherein the protein is selected from the group antibody, avidin and glucose oxidase.

6. A method according to any of claims 1 to 5, wherein the metal is gold and the gold is coated with anti-biotin antibody.

7. A method according to any of claims 1 to 6, wherein the solid support is selected from the group nitrocellulose, derivatized nylon and fluorinated polyhydrocarbons.

8. A method according to any of claims 1 to 7, wherein the solid support is in form of a filter, beads or solid sheets.

pg DNA

FIG. 1

FIG. 2a

FIG. 2b

pg DNA

MD 243

FIG. 3